(19) Europäisches Patentamt
European Patent Office
Office européen des brevets

(11) Veröffentlichungsnummer: **0 021 338**
**B1**

(12) # EUROPÄISCHE PATENTSCHRIFT

(45) Veröffentlichungstag der Patentschrift: **12.10.83**

(51) Int. Cl.³: **C 07 D 487/04,** A 61 K 31/505 // (C07D487/04, 239/00, 235/00)

(21) Anmeldenummer: **80103396.0**

(22) Anmeldetag: **18.06.80**

(54) **Neue Chinazolinderivate und pharmazeutische Präparate.**

(30) Priorität: **20.06.79 US 50395**
**08.05.80 CH 3600/80**

(43) Veröffentlichungstag der Anmeldung: **07.01.81 Patentblatt 81/1**

(45) Bekanntmachung des Hinweises auf die Patenterteilung: **12.10.83 Patentblatt 83/41**

(84) Benannte Vertragsstaaten: **AT BE CH DE FR GB IT LI LU NL SE**

(56) Entgegenhaltungen:
**EP-A-0 000 718**
**DE-A-2 305 575**
**DE-A-2 914 494**
**US-A-3 932 407**
**US-A-3 988 340**

**Die Akte enthält technische Angaben, die nach dem Eingang der Anmeldung eingereicht wurden und die nicht in dieser Patentschrift enthalten sind.**

**Chemical Abstracts, Band 87, Nr. 5, 1. August 1977, Columbus, Ohio, USA, J.S. FLEMING et al. "Effect of a new potent inhibitor of platelet aggregation (BL-3459) on experimental thrombosis" Seite 1, Abstract Nr. 33315f**

(73) Patentinhaber: **F. HOFFMANN-LA ROCHE & CO. Aktiengesellschaft, CH-4002 Basel (CH)**

(72) Erfinder: **Chodnekar, Madhukar Subraya, Dr., Rebhaldenstrasse 6, CH-4411 Seltisberg (CH)**
Erfinder: **Kaiser, Ado, Dr., Hirsweg 3, CH-4415 Lausen (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

**Chemical Abstracts, Band 91, Nr. 23, 3. Dezember 1979, Columbus, Ohio, USA, J.S. FLEMING et al. "A potent new inhibitor of platelet aggregation and experimental thrombosis, anagrelide (BL-4162A)" Seiten 50–51, Abstract Nr. 186659d**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Chinazolinderivate und pharmazeutische Präparate

Die vorliegende Erfindung betrifft neue Chinazolinderivate in D-Form oder als Racemate der Formel I

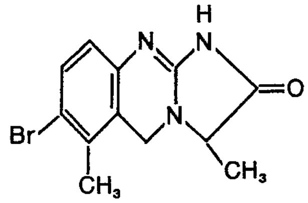

(I)

und deren Tautomere, sowie Salze solcher Verbindungen mit Mineralsäuren.

Die Gruppe der Verbindungen der Formel I besteht aus 7-Brom-1,5-dihydro-3,6-dimethyl-imidazo[2,1-b]chinazolin-2(3H)-on in D-Form oder als Racemat.

Im Gegensatz zu den im US Patent 3 932 407 beschriebenen 3-unsubstituierten Imidazochinazolinen enthalten die beanspruchten Verbindungen der Formel I einen Methylrest in 3-Stellung. Letztere Verbindungen fallen zwar unter die Formel I in der DE-A-2 832 138 (≡ EP-A-718), sie sind jedoch in dieser Offenlegungsschrift nicht spezifisch genannt und sind daher als neu anzusehen.

Die Erfindung betrifft weiterhin ein Verfahren zur Herstellung der genannten Verbindungen sowie pharmazeutische Präparate auf der Basis der genannten Verbindungen.

Die Verbindungen der Formel I können in verschiedenen tautomeren Formen vorliegen. Die Erfindung beschränkt sich daher nicht auf Verbindungen der oben dargestellten Formel I, sondern umfasst auch die Tautomeren, beispielsweise solche der Formel Ia

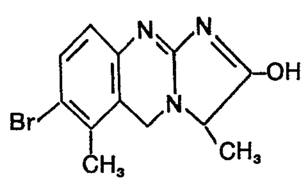

(Ia)

und Ib

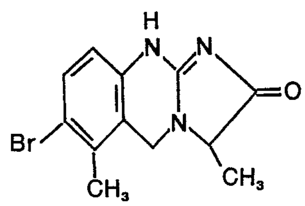

(Ib)

Die Verbindungen der Formel I und deren Tautomeren, z.B. Ia und Ib, können weiterhin in Form von Racematen oder in optisch aktiver Form vorliegen, wobei die Racemate und die Verbindungen in D-Form Gegenstand der Erfindung sind.

Beispiele physiologisch verträglicher Salze mit Mineralsäuren sind Hydrochloride, Hydrobromide, Sulfate und Phosphate.

Die Verbindungen der Formel I, deren Tautomeren und die Salze solcher Verbindungen mit Mineralsäuren können erfindungsgemäss dadurch hergestellt werden, dass man

a) eine Verbindung der Formel II

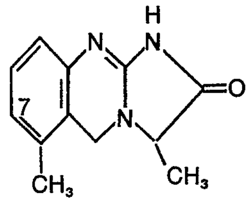

(II)

in 7-Stellung bromiert, oder

b) eine Verbindung der Formel III

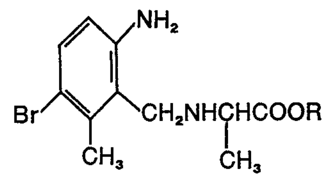

(III)

worin R nieder-Alkyl ist, mit Bromcyan umsetzt, oder

c) eine Verbindung der Formel IV

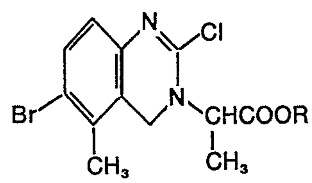

(IV)

worin R die obige Bedeutung hat, mit Ammoniak behandelt, und eine erhaltene Verbindung der Formel I oder ein Tautomer davon in dieser Form oder in Form eines Salzes mit einer Mineralsäure isoliert.

Die Bromierung einer Verbindung der Formel II in 7-Stellung wird zweckmässig in Essigsäure bei etwa Raumtemperatur durchgeführt.

Die Umsetzung einer Verbindung der Formel III mit Bromcyan wird zweckmässig unter Erwärmen in einem Lösungsmittel, wie einem niederen Alkanol, z.B. Äthanol, durchgeführt. Die Umsetzung einer Verbindung der Formel IV mit Ammoniak wird zweckmässig unter Erwärmen in einem Lösungsmittel, wie einem niederen Alkanol, z.B. Äthanol, und Wasser, durchgeführt.

Die Verbindungen der Formeln II, III und IV gehören einer allgemein bekannten Gruppe von Verbindungen an und können in Analogie zu bekannten Verbindungen, z.B. nach den in der DE-A-2 832 138 beschriebenen Methoden, hergestellt werden.

Die Verbindungen der Formel I in D-Form oder als Racemate, deren Tautomere und physiologisch verträgliche Salze solcher Verbindungen mit Mineralsäuren hemmen die Aggregation der Blutplättchen und können daher zur Verhütung von Thrombosen verwendet werden.

Die Verbindungen der Formel I in D-Form oder als Racemate und deren Tautomere können als Heilmittel, z.B. in Form pharmazeutischer Präparate, Verwendung finden, welche sie oder ihre Salze in Mischung mit einem für die enterale oder parenterale Applikation geeigneten pharmazeutischen, organischen oder anorganischen inerten Trägermaterial, wie Wasser, Gelatine, Gummi arabicum, Milchzucker, Stärke, Magnesiumstearat, Talk, pflanzliche Öle, Polyalkylenglykole, Vaseline enthalten. Die pharmazeutischen Präparate können in fester Form, z.B. als Tabletten, Dragées, Suppositorien, Kapseln, oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz-oder Emulgiermittel, Salze zur Veränderung des osmotischen Druckes oder Puffer. Sie können auch noch andere therapeutisch wertvolle Stoffe enthalten. Die orale Verabreichung der erfindungsgemässen Verbindungen ist bevorzugt. Für den Erwachsenen kommen eine orale Tagesdosis von 0,5 bis 30 mg/kg und eine parenterale Tagesdosis von 0,05 bis 10 mg/kg in Frage.

Die aggregationshemmende Wirkung wurde nach der Aggregometer-Methode von BORN [Nature 194, 927 (1962)] und MICHAL und BORN [Nature 231, 220 (1971)] nachgewiesen. Die maximale Aggregationsgeschwindigkeit wurde als Versuchsparameter genommen und die effektive Konzentration ($EC_{50}$) aus Dosis-Wirkungskurven ermittelt.

Menschliches plättchenreiches Plasma wurde aus citriertem venösem Blut durch Zentrifugation erhalten. Die Versuche wurden mit Suspensionen der Testsubstanzen in 0,9% NaCl oder mit Lösungen der Testsubstanzen in DMSO durchgeführt.

A. Suspensionen der Testsubstanzen in 0,9% NaCl.

0,18 ml Citratplasma wurden mit 10 µl Suspension der Testverbindungen versetzt, 10 Minuten bei 37 °C inkubiert, worauf die Aggregation durch Zusatz von 10 µl einer Suspension von Kollagen-Fibrillen ausgelöst wurde.

B. Lösungen der Testsubstanzen in DMSO.

0,5 ml Citratplasma wurden mit 5 µl Lösung der Testverbindung versetzt, 10 Minuten bei 37 °C inkubiert, worauf die Aggregation durch Zusatz von 5 µl einer Suspension von Kollagen-Fibrillen oder 5 µl einer $10^{-4}$ M Adenosinphosphat(ADP)-Lösung ausgelöst wurde. Als Kontrollwert diente mit DMSO inkubiertes Plasma.

Plättchenreiches Kaninchen-Plasma wurde aus mit Citrat (9 mM) versetztem arteriellem Blut durch Zentrifugieren erhalten. 0,6 ml Plasma wurde mit 5 µl Lösung der Testsubstanz versetzt und 10 Minuten bei 37 °C inkubiert, worauf die Aggregation durch Zusatz von 5 µl einer Suspension von Kollagen-Fibrillen oder 5 µl einer $10^{-4}$-M-ADP-Lösung ausgelöst wurde. Als Kontrollwert diente mit Dimethylsulfoxid inkubiertes Plasma.

Die Resultate sind in der nachstehenden Tabelle wiedergegeben.

Tabelle
Kollagen- und ADP-induzierte Plättchenaggregation

| Verbindung | Kaninchen-Plasma | | Menschliches Plasma | | |
|---|---|---|---|---|---|
| | Kollagen[1] $EC_{50}$ µM | ADP[1] $EC_{50}$ µM | Kollagen[1] $EC_{50}$ µM | ADP[1] $EC_{50}$ µM | Kollagen[2] $EC_{50}$ µM |
| D-7-Brom-1,5-dihydro-3,6-dimethyl-imidazo[2,1-b]chinazolin-2(3H)-on | | | 0,31 | | 0,98 |
| D-7-Brom-1,5-dihydro-3,6-dimethyl-imidazo[2,1-b]chinazolin-2(3H)-on-hydrochlorid | 0,78 | 0,66 | 0,50 | 1,2 | 2,5 |
| D-7-Brom-1,5-dihydro-3,6-dimethyl-imidazo[2,1-b]chinazolin-2(3H)-on-hydrobromid | | | 0,21 | | 1,3 |

[1] Lösung in DMSO
[2] Suspension in 0,9% NaCl

Beispiel 1

Eine Lösung von 52 g Brom in 200 ml Eisessig wird unter Rühren einer Suspension von 64 g D-1,5-Dihydro-3,6-dimethyl-imidazo[2,1-b]chinazolin-2(3H)-on in 400 ml Eisessig zugesetzt. Nach 2 Stunden wird das Gemisch filtriert und der Niederschlag (97,4 g, Smp. 250–255 °C) mit Eisessig gewaschen, getrocknet und aus Methanol und Diäthyläther umkristallisiert. Man erhält 64,8 g D-7-Brom-1,5-dihydro-3,6-dimethyl-imidazo[2,1-b]-chinazolin-2(3H)-on-hydrobromid, Smp. 277 bis 279 °C (Zersetzung), $[\alpha]_D^{25} = -21{,}3°$ (c = 1%, DMSO).

25 g dieses Salzes werden pulverisiert und in 2 l Wasser suspendiert. Die Suspension wird 5 Stunden gerührt und dann filtriert, der Rückstand mit Wasser gewaschen und getrocknet. Man erhält 17,25 g der freien Base, Smp. oberhalb 300 °C, $[\alpha]_D^{25} = -38°$ (in Trifluoressigsäure).

Das Ausgangsmaterial kann wie folgt hergestellt werden:

Eine Lösung von 302 g 3-Nitro-o-xylol in 200 ml Tetrachlorkohlenstoff wird unter Rühren einer Suspension von 391 g N-Bromsuccinimid und 10 g Dibenzoylperoxyd in 1000 ml Tetrachlorkohlenstoff zugefügt. Das Gemisch wird 3–4 Stunden bei Rückflusstemperatur erhitzt, dann auf Raumtemperatur abgekühlt und filtriert. Das Filtrat wird zur Trockene eingedampft. Man erhält 480 g eines Gemisches von Isomeren, 2-Methyl-6-nitrobenzylbromid und 2-Methyl-3-nitrobenzylbromid.

Eine Lösung von 900 ml Triäthylamin in 1 l Äthanol wird einer Lösung von 380 g D-Alaninäthylester-hydrochlorid in 1,5 l Äthanol zugetropft. Das auf 70 °C erwärmte Reaktionsgemisch wird mit einer Lösung der oben erwähnten 480 g Isomerengemisch in 1 l Äthanol versetzt. Das Gemisch wird 3–4 Stunden bei Rückflusstemperatur behandelt und zur Trockene eingedampft. Das Produkt wird mit 1,5 l Wasser versetzt und mit Wasser und Methylenchlorid extrahiert. Die Extrakte werden mit Wasser gewaschen, getrocknet und eingedampft. Man erhält 534 g eines Gemisches der isomeren N-(2-Methyl-6-nitrobenzyl)- und N-(2-Methyl-3-nitrobenzyl)-D-alanin-äthylester.

Eine Lösung von diesem Gemisch in 1 l Äthanol wird über 50 g eines Pd/C-Katalysators hydriert. Nach vollendeter Hydrierung wird der Katalysator abfiltriert. Dem Filtrat, enthaltend N-(2-Amino-6-methylbenzyl)- und N-(2-Amino-3-methylbenzyl)-D-alaninäthylester, werden 186 g Bromcyan zugesetzt. Das Gemisch wird 48 Stunden gerührt, dann durch Zusatz von 300 ml konzentriertem Ammoniak alkalisch gestellt und 1–2 Stunden gerührt. Der Niederschlag wird filtriert, mit Wasser gewaschen und getrocknet. Man erhält 54,4 g D-1,5-Dihydro-3,6-dimethyl-imidazo[2,1-b]-chinazolin-2(3H)-on.

Beispiel 2

In zu Beispiel 1 analoger Weise wird folgende Verbindung hergestellt:

D,L-7-Brom-1,5-dihydro-3,6-dimethyl-imidazo-[2,1-b]-chinazolin-2(3H)-on, Smp. 305 °C (Zersetzung), Smp. des Hydrobromids 272–276 °C, Smp. des Hydrochlorids: oberhalb 300 °C.

Beispiel 3

In üblicher Weise werden Tabletten folgender Zusammensetzung hergestellt:

| | |
|---|---|
| D-7-Brom-1,5-dihydro-3,6-dimethyl-imidazo-[2,1-b]chinazolin-2(3H)-on-hydrochlorid | 185,0 mg |
| Milchzucker | 15,0 mg |
| Maisstärke | 37,5 mg |
| Wasserlösliches Polyvinylpyrrolidon | 10,0 mg |
| Magnesiumstearat | 2,5 mg |
| Gesamtgewicht pro Tablette | 250,0 mg |

Beispiel 4

In üblicher Weise werden Gelatinesteckkapseln folgender Zusammensetzung hergestellt:

| | |
|---|---|
| D-7-Brom-1,5-dihydro-3,6-dimethyl-imidazo-[2,1-b]chinazolin-2(3H)-on-hydrochlorid | 200,0 mg |
| Wasserlösliches Polyvinylpyrrolidon | 2,0 mg |
| Maisstärke | 43,0 mg |
| Talk | 4,5 mg |
| Magnesiumstearat | 0,5 mg |
| Gesamtgewicht pro Kapsel | 250,0 mg |

Beispiel 5

In üblicher Weise wird eine Injektionslösung folgender Zusammensetzung hergestellt:

| | |
|---|---|
| D-7-Brom-1,5-dihydro-3,6-dimethyl-imidazo-[2,1-b]chinazolin-2(3H)-on-hydrochlorid | 114,0 mg |
| Glycerinformal | 2,4 ml |
| Wasser | 4,0 ml |

**Patentansprüche für die Vertragsstaaten: BE, CH, DE, FR, GB, IT, LI, LU, NL, SE**

1. Ein Chinazolinderivat in D-Form oder als Racemat der Formel I

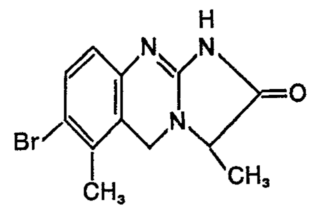

(I)

und deren Tautomere, sowie Salze solcher Verbindungen mit Mineralsäuren.

2. D,L-7-Brom-1,5-dihydro-3,6-dimethyl-imidazo[2,1-b]chinazolin-2(3H)-on, dessen Tautomere und Salze davon mit Mineralsäuren.

3. D-7-Brom-1,5-dihydro-3,6-dimethyl-imidazo-[2,1-b]-chinazolin-2(3H)-on, dessen Tautomere und Salze davon mit Mineralsäuren.

4. Verbindung nach einem der Ansprüche 1–3 zur Verwendung als die Blutplättchenaggregation hemmender Wirkstoff.

5. Arzneimittel enthaltend eine Verbindung nach einem der Ansprüche 1–3.

**Patentansprüche für den Vertragsstaat: AT**

1. Verfahren zur Herstellung eines Chinazolinderivates in D-Form oder als Racemat und der Formel I

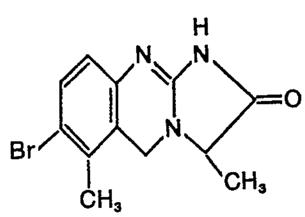

(I)

und deren Tautomeren, sowie von Salzen solcher Verbindungen mit Mineralsäuren, dadurch gekennzeichnet, dass man

a) eine Verbindung der Formel II

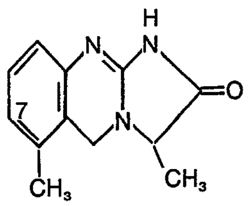

(II)

in 7-Stellung bromiert, oder

b) eine Verbindung der Formel III

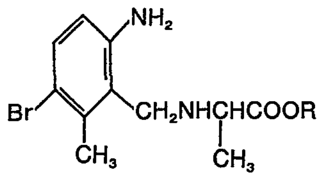

(III)

worin R nieder-Alkyl ist, mit Bromcyan umsetzt, oder

c) eine Verbindung der Formel IV

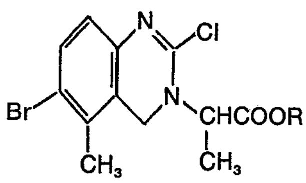

(IV)

worin R die obige Bedeutung hat, mit Ammoniak behandelt und eine erhaltene Verbindung der Formel I oder ein Tautomer davon in dieser Form oder in Form eines Salzes mit einer Mineralsäure isoliert.

2. Verfahren nach Anspruch 1, dadurch gekennzeichnet, dass man DL-7-Brom-1,5-dihydro-3,6-dimethyl-imidazo[2,1-b]chinazolin-2(3H)-on, dessen Tautomere oder Salze davon mit Mineralsäuren herstellt.

3. Verfahren nach Anspruch 1, dadurch gekennzeichnet, dass man D-7-Brom-1,5-dihydro-3,6-dimethyl-imidazo[2,1-b]chinazolin-2(3H)-on, dessen Tautomere oder Salze davon mit Mineralsäuren herstellt.

**Claims for the contracting States: BE, CH, DE, FR, GB, IT, LI, LU, NL, SE:**

1. A quinazoline derivative of formula I

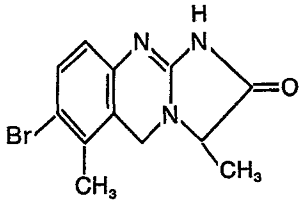

(I)

in D-form or as the racemate, their tautomers and salts of such compounds with mineral acids.

2. D,L-7-bromo-1,5-dihydro-3,6-dimethyl-imidazo[2,1-b]quinazolin-2(3H)-one, its tautomers and salts thereof with mineral acids.

3. D-7-bromo-1,5-dihydro-3,6-dimethyl-imidazo[2,1-b]quinazolin-2(3H)-one, its tautomers and salts thereof with mineral acids.

4. Compound according to anyone of claims 1–3 for use as an active substance which inhibits the aggregation of blood platelets.

5. Medicament containing a compound according to any one of claims 1–3.

**Claims for the contracting State: AT**

1. Process for the manufacture of a quinazoline derivative of formula I

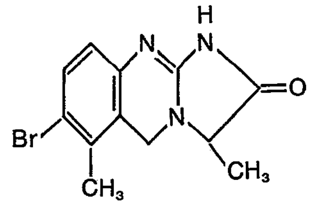

(I)

in D-form or as the racemate, and their tautomers as well as of salts of such compounds with mineral acids, characterized by

a) brominating a compound of formula II

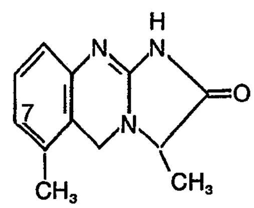

(II)

in the 7-position, or

b) reacting a compound of formula III

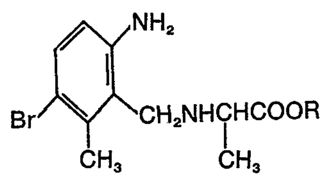

(III)

wherein R is lower-alkyl, with cyanogen bromide, or

c) treating a compound of formula IV

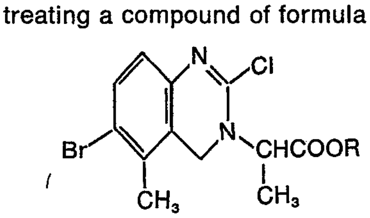

(IV)

wherein R has the above significance, with ammonia and isolating an obtained compound of formula I or a tautomer thereof in that form or in the form of a salt with a mineral acid.

2. Process according to claim 1, characterized in that DL-7-bromo-1,5-dihydro-3,6-dimethyl-imidazo[2,1-b]quinazolin-2(3H)-one, its tautomers or salts thereof with mineral acids are manufactured.

3. Process according to claim 1, characterized in that D-7-bromo-1,5-dihydro-3,6-dimethyl-imid-

azo[2,1-b]quinazolin-2(3H)-one, its tautomers or salts thereof with mineral acids are manufactured.

**Revendications pour les Etats contractants: BE, CH, DE, FR, GB, IT, LI, LU, NL, SE**

1. Un dérivé de quinazoline sous la forme D ou à l'état de racémate de formule I:

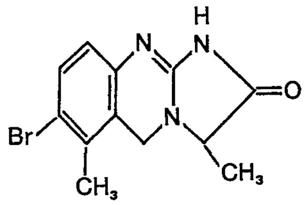

(I)

et ses tautomères, ainsi que les sels de ces composés et d'acides minéraux.

2. La D,L-7-bromo-1,5-dihydro-3,6-diméthyl-imidazo[2,1-b]quinazoline-2(3H)-one, ses tautomères et leurs sels d'acides minéraux.

3. La D-7-bromo-1,5-dihydro-3,6-diméthyl-imidazo[2,1-b]quinazoline-2(3H)-one, ses tautomères et leurs sels d'acides minéraux.

4. Composé selon l'une des revendications 1 à 3, pour l'utilisation en tant que substance active inhibant l'agglutination des plaquettes du sang.

5. Médicament contenant un composé selon l'une des revendications 1 à 3.

**Revendications pour l'Etat contractant: AT**

1. Procédé de préparation d'un dérivé de quinazoline sous la forme D ou à l'état de racémate et de formule I:

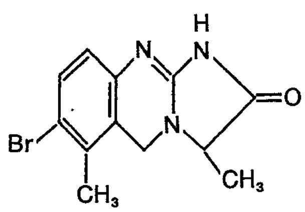

(I)

et de leurs tautomères, ainsi que des sels de ces composés et d'acides minéraux, caractérisé en ce que:

a) on brome en position 7 un composé de formule II

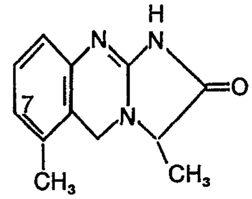

(II)

ou bien

b) on fait réagir un composé de formule III:

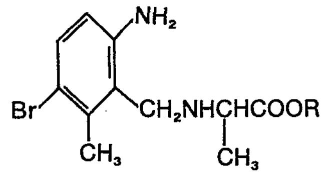

(III)

dans laquelle R représente un groupe alkyle inférieur, avec le bromure de cyanogène, ou bien

c) on traite par l'ammoniac un composé de formule IV

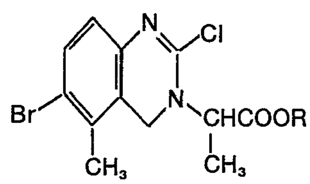

(IV)

dans laquelle R a la signification indiqué ci-dessus, et on isole un composé de formule I ou un tautomère de ce composé sous cette forme ou sous forme d'un sel d'acide minéral.

2. Procédé selon la revendication 1, caractérisé en ce que l'on prépare la DL-7-bromo-1,5-dihydro-3,6-diméthyl-imidazo[2,1-b]quinazoline-2(3H)-one, ses tautomères ou leurs sels d'acides minéraux.

3. Procédé selon la revendication 1, caractérisé en ce que l'on prépare la D-7-bromo-1,5-dihydro-3,6-diméthyl-imidazo[2,1-b]quinazoline-2(3H)-one, ses tautomères ou leurs sels d'acides minéraux.